(19) Europäisches Patentamt
European Patent Office
Office européen des brevets

(11) Publication number: **0 175 330 B1**

(12) # EUROPEAN PATENT SPECIFICATION

(45) Date of publication of patent specification: **27.11.91** (51) Int. Cl.$^5$: **G01N 33/72**, G01N 33/52

(21) Application number: **85111696.2**

(22) Date of filing: **16.09.85**

(54) **Integral multilayer analytical element for quantitative analysis of bilirubin.**

(30) Priority: **17.09.84 JP 194289/84**

(43) Date of publication of application:
**26.03.86 Bulletin 86/13**

(45) Publication of the grant of the patent:
**27.11.91 Bulletin 91/48**

(84) Designated Contracting States:
**DE GB**

(56) References cited:

| | |
|---|---|
| EP-A- 0 019 147 | EP-A- 0 025 889 |
| EP-A- 0 087 150 | EP-A- 0 087 599 |
| EP-A- 0 115 873 | EP-A- 0 119 861 |
| GB-A- 2 085 581 | US-A- 4 274 832 |

(73) Proprietor: **FUJI PHOTO FILM CO., LTD.**
**210 Nakanuma Minami Ashigara-shi**
**Kanagawa 250-01(JP)**

(72) Inventor: **Nagatomo, Shigeru**
**c/o Fuji Photo Film Co. Ltd., 3-11-46, Senzui**
**Asaka-shi, Saitama(JP)**
Inventor: **Kageyama, Shigeki**
**c/o Fuji Photo Film Co. Ltd., 3-11-46, Senzui**
**Asaka-shi, Saitama(JP)**
Inventor: **Yoshida, Takashi**
**c/o Fuji Photo Film Co. Ltd., 3-11-46, Senzui**
**Asaka-shi, Saitama(JP)**
Inventor: **Miyazako, Takushi**
**c/o Fuji Photo Film Co. Ltd., 3-11-46, Senzui**
**Asaka-shi, Saitama(JP)**
Inventor: **Osada, Chiaki**
**c/o Fuji Photo Film Co. Ltd., 3-11-46, Senzui**
**Asaka-shi, Saitama(JP)**
Inventor: **Katsuyama, Harumi**
**c/o Fuji Photo Film Co. Ltd., 3-11-46, Senzui**
**Asaka-shi, Saitama(JP)**

(74) Representative: **Kraus, Walter, Dr. et al**
**Patentanwälte Kraus, Weisert & Partner**
**Thomas-Wimmer-Ring 15**
**W-8000 München 22(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to an integral multilayer analytical element for quantitative analysis of bilirubin, and more particularly to an integral multilayer analytical element employable for quantitative analysis of bilirubin based on a dry system with high accuracy by a simple and quick operation.

Bilirubin, a principal component of a bile pigment in a body fluid, is produced in serum by decomposition of heme originating from hemoglobin in red blood corpuscles. Bilirubin is then absorbed by the liver, in which bilirubin is converted to e.g. a glucuronic acid-conjugated product and excreted in bile. The content of bilirubin in blood increases in response to increase of decomposition of hemoglobin as well as decrease of liver function. Accordingly, the quantitative analysis of bilirubin is considered to be an indispensable test item in the clinical test.

The colorimetric quantitative analysis of red azobilirubin produced by coupling reaction of bilirubin and diazotized sulfanilate (p-sulfobenzenediazonium salt, Ehrlich reagent), based on the Ehrlich reaction discovered by A. A. Hijmans van den Bergh, is known as a method for the quantitative analysis of bilirubin in serum. This method is generally named a diazo method.

Details of methods for quantitative analysis of bilirubin in serum are described in the following publications;

M. Michaelsson: Scan. J. Clin. Lab. Invenst.: 13(Supple.), 1 - 80(1961);

H. Malloy: J. Biol. Chem., 119, 481(1939);

Z. K. Shihabi et al.: American Journal of Medical Technology, 43(10), 1004 - 1007(1977);

"Comprehensive Text of Clinical Test Technology" edited by Ishii, Vol. 6, pp. 332-350 (Igaku Shoin, 1975); and,

"Clinical Chemical Analysis II, Nitrogenous Component" edited by the Analytical Chemical Society of Japan, 2nd edition, pp. 248-279 (Tokyo Kagaku Dojin, 1979).

A great number of analytical processes for quantitative analysis of bilirubin based on the above-mentioned diazo method employing a dry analytical element have been proposed, for instance, in Japanese Patent Publication No. 53(1978)-28119, and Japanese Patent Provisional Publications Nos. 54(1979)-33094, 55(1980)-44992, 56(1981)-10255, 56(1981)-30503, and 57(1982)-23859.

On the other hand, a number of studies on improvement of the analytical accuracy of a multilayer analytical element for quantitative analysis of bilirubin by way of utilizing a non-fibrous isotropically porous spreading layer have been already made and described in Japanese Patent Publication No. 57(1982)-25783 and Japanese Patent Provisional Publications Nos. 57(1982)-37262 and 57(1982)-110942. It is noted, however, that all of the multilayer analytical elements for quantitative analyis of bilirubin employing an isotropically porous spreading layer described in these publications are not based on the above-mentioned diazo method, but based on other quantitative analytical systems.

A multilayer analytical element based on the diazo method is thought to have some drawbacks described below.

First, non-diffusive bilirubins such as protein-conjugated bilirubin is difficult to detect, and accordingly the total bilirubin content is not appropriately analyzed by the analytical element.

Bilirubin is generally present in combined form with a protein (e.g. serum albumin) in a liquid sample. If an analytical element, having a polymer film which disturbs permeation of the protein laminated thereon, is employed for the analysis, the liquid sample is required to be processed with a dissociating agent such as caffeine, dyphylline (C. A. Registory No [479-18-5]), sodium benzoate, or sodium acetate for separating bilirubin from the protein in advance of the analysis. Accordingly, in order to quantitatively analyze the bilirubin combined with a protein, such bilirubin should sufficiently migrate or diffuse into a reagent layer containing a detection reagent, so that it can be detectable upon reaction with the detection reagent.

For this reason, the conventional integral multilayer analytical element for this purpose contains the dissociating agent in the spreading layer. Nevertheless, the dissociating agent is not effective for dissociating a bilirubin conjugated with albumin through covalent bonding, which has a comparatively large molecular weight and a hydrophobic molecular structure. Therefore the migration and diffusion of the conjugated bilirubin cannot be enhanced in such case.

Secondly, stability of the diazonium salt is low, and accordingly preservability of the multilayer analytical element employing diazonium salt is insufficient.

EP-A-0 119 861 - constituting a prior art document in the sense of Article 54(3) EPC - describes a multilayer analytical element for quantitative analysis of bilirubin in a liquid sample by a diazo method, which comprises a laminated structure consisting essentially of: (1) a porous reagent layer containing an acidic binder and a diazonium salt being reactive to bilirubin to produce azobilirubin; (2) an absorbent layer capable of absorbing an excessive liquid portion; and (3) a liquid-impermeable light-transmissive support.

The object of the present invention is to provide an integral multilayer analytical element for quantitative analysis of bilirubin which enables a non-migratory non-diffusive bilirubin to sufficiently diffuse within a porous reagent layer.

In said integral multilayer analytical element the stability and preservability of diazonium salt should be improved.

There is provided by the present invention an integral multilayer analytical element for quantitative analysis of bilirubin in a liquid sample, which comprises:

(I) a porous reagent layer which contains (1) a polymer having a sulfonic acid group in its repeating unit, (2) an arylsulfonic acid, and (3) a diazonium salt being reactive to bilirubin to produce azobilirubin;

(II) an absorbent layer capable of absorbing an excessive liquid portion; and

(III) a liquid-impermeable light-transmissive support.

According to the present invention, the drawbacks of the conventional analytical element brought about by the non-diffusiveness of the analyte is obviated by the provision of the absorbent layer to the analytical element, thereby making it possible to detect a non-migratory non-diffusive bilirubin. The absorbent layer of the invention serves to absorb a solvent (i.e., liquid medium) of a liquid sample without allowing bilirubin in the liquid sample to elevate the concentration of the bilirubin diffusing into the reagent layer, thereby enhancing the efficiency of the reaction between the non-migratory non-diffusive bilirubin and a detection reagent.

Further the porous reagent layer of this invention contains a polymer having a sulfonic acid group in the repeating units, so that the stability and preservability of diazonium salt is improved. The polymer having a sulfonic acid group in the repeating unit is such an acidic binder that it effectively protects the diazonium salt.

The porous reagent layer of the invention can be prepared by superposing, for instance, through pressing, the material of the porous reagent layer having the polymer and diazonium salt on the absorbent layer provided on a transparent support, to obtain an integrated structure.

The diazonium salt, acidic binder and other additives can be incorporated into the porous reagent layer, for instance, by immersing the porous material into a solution containing these substances or by coating the solution substances over the porous material, and then drying the material. Otherwise, the solution containing these substances can be coated on a reagent layer integrally combined with an absorbent layer on a support, whereafter the so-treated integral element is dried.

The material constituting the matrix of the porous reagent layer of the invention can be a material having voids allowing easy migration or diffusion of a high molecular weight analyte in and through the material. Examples of the material include filter paper, non-woven fabric, woven fabric, knitted cloth, glass fiber sheet (e.g., glass fiber filter), and membrane filter formed of blushed polymer.

Examples of the woven fabrics (woven cloth) which can be used for the porous reagent layer include those disclosed in Japanese Patent Provisional Publications No. 55(1980)-164356 and No. 57(1982)-66359. Among the woven fabrics, plain weave fabrics made of warp and weft are preferred. Among plain woven fabrics, thin cloth, muslin, broadcloth and poplin are preferred.

Examples of yarns for woven cloth include those composed of the same materials as those constituting knitted cloths as described in more detail hereinafter. Any of filament yarn and spun yarn (twist yarn) can be used, and the spun yarn is preferred. The yarn diameter of the woven fabric is generally in the range of about 20S to about 150S, preferably about 40S to about 120S in terms of cotton spinning yarn count or in the range of about 35 to about 300D, preferably about 45 to about 130D in terms of silk thread denier. The thickness of the woven fabric is generally in the range of about 100 to about 500 $\mu$m, preferably about 120 to 350 $\mu$m. The voids of the woven fabric are generally in the range of about 40 to about 90%, preferably about 50 to about 85%.

Examples of the knitted fabrics which can be used for the porous reagent layer include many kinds of knitted fabrics, among which warp knitted fabric and weft knitted fabric are preferred. Examples of the warp knitted fabrics include single atlas knitted cloth, tricot knitted cloth, double tricot knitted cloth, milanese knitted cloth and rashar knitted cloth. Examples of the weft knitted fabrics include plain weave knitted cloth, pearl knitted cloth, rib stitch cloth, and double face knitted cloth. Examples of the yarns for knitted fabrics include yarns of natural fibers such as cotton, silk and wool; yarns composed of fine fibers or single fibers of regenerated cellulose (e.g. viscose rayon and cupra), semi-synthetic organic polymer (e.g. cellulose diacetate and cellulose triacetate), synthetic organic polymer (e.g. polyamide such as nylon, acetalated polyvinyl alcohol such as vinylon, polyacrylonitrile, polyethylene terephthalate, polyethylene, polypropylene and polyurethane), and yarns composed of fiber blends of a natural fiber and a regenerated cellulose or a semi-synthetic or synthetic organic polymer fiber. Any of filament yarn and spun yarn can be used, and spun yarn is preferred. The diameter of the yarn for knitted fabric is generally in the range of from about 40

to 150S, preferably about 60 to about 120S in terms of cotton spinning yarn count, or in the range of about 35 to about 130D, preferably about 45 to about 90D in terms of silk thread denier. The number of knitting gauge of the knitted fabric is generally in the range of about 20 to about 50. The thickness of the knitted fabric is generally in the range of about 100 to about 600 µm, preferably about 150 to about 400 µm. The voids of the knitted fabric are generally in the range of about 40 to about 90%, preferably about 50 to about 85%. The warp knitted fabric, tricot knitted cloth, rashar knitted cloth, milanese knitted cloth and double tricot knitted cloth are preferred, because shrinkage in the wale's direction is small, the operation in the lamination stage of knitted goods is easy and the stitches are not easily loosened during cutting.

The material employable for forming the matrix of the porous reagent layer of the invention is not restricted by the above-described materials. For instance, non-fabric isotropically porous layer prepared by a coating method described in Japanese Patent Publication No. 53(1978)-21677 and U.S. Patent 3,992,158 can be used.

Examples of the diazonium salt employable as the bilirubin detection reagent include a variety of diazonium salts being conventionally known in the art of quantitative analysis of bilirubin utilizing the wet and dry diazo methods. Among these diazonium salts, a stable diazonium salt is preferred. Examples of the stable diazonium salt include dichlorobenzenediazonium salt, sulfobenzenediazonium salt, N-substituted sulfoneamidebenzenediazonium salt, dichloronitrobenzene diazonium salt, substituted biphenylbisdiazonium salt and an aryl diazonium salt disclosed in Japanese Patent Appliraction No. 58(1983)-45547 which contains an alkoxycarbonyl group, alkylaminosulfonyl group or alkylaminocarbonyl group. Further the above diazonium salts are preferably stabilized by way of utilizing a counter ion such as arylsulfonate, tetrafluoroborate, hexafluorophosphate etc.

The polymer having a sulfonic acid group in the repeating unit is preferably a polymer in which at least 10 mol% (more preferably at least 30 mol%) of the repeating units contain a sulfoalkyl group or a sulfophenyl group.

An example of the repeating unit which contains the sulfoalkyl group has the formula (I):

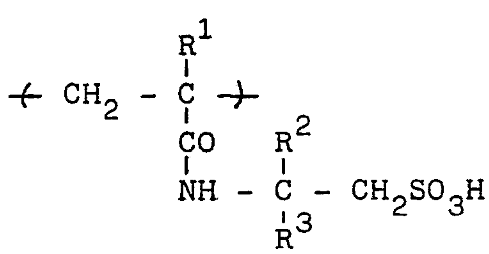

(I)

in which $R^1$ and $R^2$, which may be identical or different, represent hydrogen atom or methyl group; and $R^3$ represents an alkyl group containing 1 - 18 carbon atoms, an aryl- or alkyl-substituted aryl group containing 6 - 10 carbon atoms, or an alkoxycarbonyl group containing 1 - 5 carbon atoms.

$R^3$ in the formula (1) is preferably an alkyl group containing 1 - 8 carbon atoms, phenyl group, or methoxycarbonyl group, more preferably methyl group, ethyl group or propyl group.

A monomer which gives the repeating unit represented in the formula (I) may be prepared by a known method or a similar method. For example, the preparation of the monomer in which $R^1$ is hydrogen atom, is described in U.S. Patent 3,506,707.

Examples of the monomer which give the repeating unit represented in the formula (I) include N-(sulfoalkyl)acrylamide [preferably, N-(β-sulfo-t-butyl)acrylamide] and N-(sulfoalkyl)methacrylamide [preferrably, N-(β-sulfo-t-butyl)methacrylamide].

Examples of the monomer having sulfophenyl group in the repeating unit include p-styrene sulfonate.

The polymer having a sulfoalkyl group in the repeating units may be a copolymer between a monomer of the formula (I) and other monomer which can be copolymerized with the former monomer. Examples of the latter monomer include an olefin (e.g., ethylene, propylene etc.); acrylic acid and its derivatives, such as acrylic acid, acrylate ester (e.g., methyl ester, ethyl ester), acrylate hydroxyalkyl ester (e.g., hydroxyethyl ester) and acrylamide; methacrylic acid and its derivatives, such as methacrylic acid, methacrylate ester (e.g., methyl ester, ethyl ester, butyl ester, isobutyl ester, hexyl ester), methacryl amide and methacrylate hydroxyalkyl ester (e.g., hydroxyethyl ester); and unsaturated dicarboxylic acid, such as maleic acid and fumaric acid.

The polymer may be a homopolymer. Examples of the homopolymer include poly-2-acrylamide-2-methylpropanesulfonate and poly-2-methacrylamide-2-methylpropanesulfonate.

These polymers having a sulfonic acid group in the repeating units can be easily prepared referring to the following publications;

"Synthesis of Polymer I" edited by C. G. Overberger; translated by T. Ohtsu and M. Kinoshita (Kagaku Dojin, 1972).

The polymers can be used in the form of the mixture with other water-soluble polymer. Examples of the other water-soluble polymer include polyacrylamide, poly-n-vinylpyrrolidone, hydroxyethylcellulose, hydroxypropylcellulose, ethylcellulose, carboxymethylcellulose and agarose.

The polymers containing a sulfonic acid group in the repeating units may be also used in the form of a mixture of the polymer and an aqueous latex of a hydrophobic polymer.

An aryl sulfonic acid can be optionally incorporated in the porous reagent layer of this invention. Examples of the aryl sulfonic acid include benzenesulfonic acid and its derivatives such as benzenesulfonic acid, toluenesulfonic acid, xylenesulfonic acid and sulfosalicylic acid; and naphthalenesulfonic acid. In these aryl sulfonic acids, the substituted position of the sulfo group is not restricted.

It is supposed that the aryl sulfonic acid has some effects such as stabilizing (preventing decomposition of) the diazonium salt, adjusting or buffering pH by its own acidity and promoting condensation reaction between bilirubin and diazonium salt. Accordingly the aryl sulfonic acid can be utilized to improve or complement the effect of the polymer containing a sulfonic acid group in the repeating unit. Thus, the aryl sulfonic acid content may be determined according to the effect of the said polymer, but in general the content is in the range of about 7 to about 70 times (preferably, about 10 to about 50 times) as much as the diazonium compound content by weight ratio.

Light-blocking fine particles can be also optionally incorporated in the porous reagent layer of this invention. Examples of the light-blocking fine particles include white particles such as titanium dioxide fine particles (rutile-type or anatase-type) or barium sulfate particles.

The light-blocking fine particles are used to enhance the stability of white-reflection density of azobilirubin produced in proportion to the bilirubin density, when the reflection density is measured from the side of the transparent support by means of reflection photometry. Further, the light-blocking particles in this integral multilayer analytical element for quantitative analysis of bilirubin have another effect to assure metering effect in wide range of the quantity of the sample spotted and supplied. The metering effect is a function capable of spreading a liquid sample (an aqueous liquid sample such as whole blood, blood plasma, serum, urine etc.) in such a manner that the spread area of the liquid is approximately in proportion to the amount of the liquid.

The porous reagent layer of the invention can contain a diazo-coupling accelerater, a surface active agent, and/or other additives, in addition to the diazonium salt, the polymer having a sulfonic acid group, the aryl sulfonic acid and the light-blocking fine particles.

The porous reagent layer of the invention preferably contains a reaction accelerater to accelerate the reaction between the analyte and the diazonium salt.

The reactivity between bilirubin and a diazonium salt is prominently influenced by the solubility of the bilirubin. Since the free bilirubin (indirect bilirubin) of non-conjugate type is highly hydrophobic and sparingly water-soluble, the rate of reaction with a diazonium is low. In contrast, since the conjugate bilirubin and albumin-conjugated bilirubin are highly soluble in water, these rapidly reacts with a diazonium salt. Accordingly, the difference on the reaction rate residing between a variety of bilirubins is utilized to analyze separately each of the direct bilirubin (conjugate or protein-conjugated bilirubin) and the indirect bilirubin (free bilirubin).

The analytical element of the present invention can be employed in the same manner to separately analyze each of the direct and indirect bilirubins.

In the quantitative analysis of the total bilirubin content or a quick analysis of the indirect bilirubin, a reaction accelerator is necessarily included in the analytical element. The reaction accelerator employable for this purpose is known and described in a variety of texts. Examples of the accelerator include alcohols (e.g., methanol, ethanol, etc.), caffeine, sodium benzoate, sodium acetate, dyphylline (C. A. Registory No. [479-18-5]), urea, a nonionic surfactant, gum arabic, an acid amide, sulfoxide, etc. The reaction accelerator can be optionally incorporated into the analytical element of the present invention.

The absorbent layer of this invention is constituted by a material comprising a hydrophilic polymer as a main component. The hydrophilic polymer preferably contains a group capable of attracting and retaining the introduced water or aqueous medium within the absorbent layer through strong hydrogen bond, preferably has a degree of swelling at a high level, and is preferably water-insoluble.

The degree of swelling of the absorbent layer preferably ranges from approx. 1.5 to approx. 30 times, more preferably from approx. 2.5 to approx. 20, as much as the orignal volume. In this specification, the term "degree of swelling" means a ratio of thickness of a wetted layer to a dry layer, in which the thickness

of the wetted layer is measured upon reaching an equilibrium state after swelling the dry layer with water at 30°C on a polyethylene terephthalate support.

The hydrophilic polymer employable for the formation of or incorporation into the absorbent layer can be a non-porous polymer capable of absorbing and retaining water because of its swelling property or other properties, or a polymer having cross-linked molecular chains capable of absorbing and retaining water of its microporous structure showing a swelling property.

Examples of the hydrophilic polymer satisfying the above-described conditions include natural polymers or their crosslinked products such as gelatin, dextran, starch, and modified starch; homopolymers and copolymers of hydrophilic monomers such as hydroxyethyl acrylate, hydroxyethyl methacrylate, N-(hydroxyalkyl)acrylámide (e.g., N-(3-hydroxy)acrylamide, etc.); polyvinyl alcohol; and copolymers of the acrylamide with one or more of the above-mentioned monomers, in which the homopolymer and copolymer can be modified through crosslinking. Most preferred is gelatin.

The swelling degrees of some hydrophilic polymers are given below.

| | |
|---|---|
| Unhardened gelatin | approx. 6 - 7 |
| Hardened gelatin | approx. 3 - 4 |
| hydroxyethyl-modified agarose | approx. 1.5 |
| Dextran | approx. 2 |
| Crosslinked polyacrylamide/agarose | approx. 17 |
| Polyhydroxyethyl acrylate/ acrylamide gel | approx. 15 |
| Crosslinked or non-crosslinked polyvinyl alcohol | approx. 3 - 15 |

The thickness of the absorbent layer preferably ranges from 3 to 50 μm, most preferably from 10 to 30 μm. Not only single absorbent layer but also plural absorbent layers can be constituted.

There is no spcecific limitation on the support employable in the invention, so far as it allows transmission of light but not allows permeation of water. Such support is already known in the art. Examples of the support include transparent supports made of glass plate or high-transparent synthetic resins such as polyethylene terephthalate, polycarbonate of bisphenol A, polystyrene, cellulose esters and polyacrylates. The thickness of the support generally ranges from approx. 50 μm to approx. 2 mm, preferably from approx. 80 μm to approx. 300 μm.

As described hereinbefore, the integral multilayer analytical element of the present invention comprises the laminated structure of the porous reagent layer, the absorbent layer, and the support. These layers are preferably laminated in the order mentioned above. Further, the integral multilayer analytical element can further have one or more functional layers such as a spreading layer, an analyte (bilirubin, a conjugated complex between bilirubin and other substance, etc.) diffusion-preventing layer, or a light-blocking layer. Accordingly, the integral multilayer analytical elements having such other layers are also embodiments of the invention.

The present invention is further described by the following examples.

EXAMPLE 1

(1) Support;

Colorless transparent polyethylene terephthalate (PET) film (thickness: 180 μm)

(2) Absorbent Layer-1;

This coating solution (I) was coated over the support and dried to form an absorbent layer-1 (thickness: 15 μm, dry basis).

Coating Solution (I)

| | |
|---|---|
| Polyvinyl alcohol (a degree of saponification: 88%, viscosity of 4% aqueous solution at 20 °C: 5 cps) | 100 g. |
| 50 % Aqueous solution of poly(2-hydxoy-3-oxypropylene)-n-nonylphenyl ether | 5 g. |
| Water | 895 mℓ |

(3) Absorbent Layer-2;

The following coating solution (II) was coated over the absorbent layer-1 and dried to form an absorbent layer-2 (thickness: 10 µm, dry basis).

Coating Solution (II)

| | |
|---|---|
| Polyvinyl alcohol (saponification ratio: 99%, viscosity in 4% aqueous solution at 20 °C: 30 cps) | 80 g. |
| 50 % Aqueous poly(2-hydxoy-3-oxypropylene)-n-nonylphenyl ether solution | 5 g. |
| Water | 915 mℓ |

(4) A cotton cloth (blood woven cloth woven made of No. 100 count two folded yarn) treated with a surfactant was wet-laminated on the absorbent layer-2 to form a matrix of a porous reagent layer.

(5) The matrix of the porous reagent layer was coated and thereby impregnated with the following coating solution (III).

Coating Solution (III)

| | |
|---|---|
| 7-(2,3-Dihydroxypropyl)theophylline [479-18-5] | 150 g. |
| Sulfosalicylic acid | 30 g. |
| Hydroxypropyl methacrylate-N-(α-sulfomethyl-α-methylethyl) acrylamide (6:4) copolymer (1.5 % aqueous solution) | 1,000 g. |
| 2,4-Dichlorobenzene diazonium sulfosalicylate | 1.9 g. |

(6) Further, the matrix was coated and thereby impregnated with the following coating solution (IV).

Coating Solution (IV)

| | |
|---|---|
| Titanium dioxide fine particles | 55 g. |
| 7-(2,3-Dihydroxypropyl)theophylline [479-18-5] | 150 g. |
| 50% Aqueous solution of poly(2-hydroxy-3-oxypropyl)-n-nonylphenyl ether | 10 g. |
| Water | 1,000 mℓ |

Thus an integal multilayer analytical element for quantitative analysis of bilirubin according to this invention was prepared.

The above-prepared analytical element was cut into a square piece (15 mm x 15 mm), and inserted in a plastic mount in the manner as described in Japanese Patent Provisional Publication No. 57(1982)-63452, to give an analytical slide for quantitative analysis of bilirubin.

On the cotton cloths containing a diazonim salt (porous reagent layer) of the analytical slides were applied 10 μℓ of a control serum (various bilirubin content) per each. The slides were incubated at 37°C for 6 hours, and then measured on the color density formed thereon from the support side through a green filter at wavelength of 540 nm. The results are set forth in Table 1.

Table 1: Reflection Density of Analytical Element (OD)

| Bilirubin content in control serum (mg/dℓ) | | | | |
|---|---|---|---|---|
| 0 | 5.0 | 10.0 | 15.0 | 20.0 |
| 0.225 | 0.375 | 0.500 | 0.610 | 0.665 |

EXAMPLE 2 (Comparative Example)

The procedure of Example 1 was repeated except that the sulfosalicylic acid was not employed in the coating solution (III), to prepare an analytical slide for quantitative analysis of bilirubin. The slide was measured on the color density in the same manner as in Example 1. The results are set forth in Table 2.

Table 2: Reflection Density of Analytical Element (OD)

| Bilirubin content in control serum (mg/dℓ) | | | | |
|---|---|---|---|---|
| 0 | 5.0 | 10.0 | 15.0 | 20.0 |
| 0.225 | 0.286 | 0.348 | 0.399 | 0.435 |

EXAMPLE 3

The procedure of Example 1 was repeated except that the sulfosalicylic acid was replaced with 45 g. of benzenesulfonic acid in the coating solution (III), to prepare a slide for quantitative analysis of bilirubin. The slide was measured in the same manner as in Example 1. The results showed that difference between reflection densities in 20 mg/dℓ and 0 mg/dℓ of bilirubin content was 0.35.

EXAMPLE 4

The procedure of Example 3 was repeated exept that 90 g. of benzenesulfonic acid was employed in the coating solution (III), to prepare an analytical slide for quantitative analysis of bilirubin. The slide was measured in the same manner as in Example 1. The results showed that the difference between reflection densities in 20 mg/dℓ and 0 mg/dℓ of bilirubin content was 0.39.

EXAMPLE 5 (Comparative Example)

The procedure of Example 1 was repeated except that the sulfosalicylic acid was replaced with the equivalent mole of malic acid or tartaric acid in the coating solution (III), to prepare an analytical element for quantitaitive analysis of bilirubin. Preservability of the diazonium salt in each analytical element (including Examples 1 to 5) was tested for 10 days at 45 °C. In the element using an aryl sulfonic acid, not less than 80 % of the diazonium salt remained. On the other hand, in the element using malic acid or tartaric acid, 10 % to 20 % of the diazonium salt remained.

EXAMPLE 6

The procedure of Example 1 was repeated except that the titanium dioxide fine particles were not employed in the coating solution (IV), to prepare an analytical slide for quantitative analysis of bilirubin.

To the slides (Examples 1 and 6) were applied various amounts of a control serum (bilirubin content: 15 mg/dℓ) per each. The slides were then measured on the color density in the same manner as in Example 1. The results are set forth in Table 3.

Table 3

| Reflection Density of Analytical Element (OD) | | |
|---|---|---|
| Amount of spotted sample | $TiO_2$ (Example 1) | $TiO_2$ free (Example 6) |
| 6 $\mu\ell$ | 0.590 | 0.644 |
| 8 | 0.600 | 0.667 |
| 10 | 0.601 | 0.685 |
| 12 | 0.602 | 0.694 |
| 14 | 0.601 | 0.702 |

The result of the Table 3 showed that the reflection density of the analytical element containing the titanium dioxide fine particles was much less affected by the amount of the spotted sample. Thus, the element containing the titanium dioxide fine particles showed an excellent metering effect.

## Claims

1. An integral multilayer analytical element for quantitative analysis of bilirubin in a liquid sample, which comprises:
   (I) a porous reagent layer which contains (1) a polymer having a sulfonic acid group in its repeating unit, (2) an arylsulfonic acid, and (3) a diazonium salt being reactive to bilirubin to produce azobilirubin;
   (II) an absorbent layer capable of absorbing an excessive liquid portion; and
   (III) a liquid-impermeable light-transmissive support.

2. The analytical element claimed in claim 1, in which said polymer contains the repeating units having a sulfonic acid group in a ratio of at least 10 mol.% based on the whole repeating units constituting said polymer.

3. The analytical element claimed in claim 1, in which the content of said arylsulfonic acid is in the range of 7 to 70 times as much as the diazonium salt content by weight ratio.

4. The analytical element claimed in claim 1 or 2, in which said porous reagent layer contains light-blocking fine particles.

5. The analytical element claimed in claim 4, in which said light-blocking fine particles are titanium dioxide fine particles.

6. The analytical element claimed in claim 1 or 2, in which said porous reagent layer has a matrix selected from the group consisting of woven fabric and knitted fabric.

7. The analytical element claimed in claim 1 or 2, in which said absorbent layer comprises a hydrophilic polymer having a degree of swelling in water at 30°C in the range of 1.5 to 30 times as much as the original volume.

8. The analytical element claimed in claim 1 or 2, in which a layer for preventing diffusion of analyte is provided between the porous reagent layer and the absorbent layer.

## Revendications

1. Elément d'analyse multicouche intégral pour l'analyse quantitative de la bilirubine dans un échantillon liquide, qui comprend :
   (I) une couche réactive poreuse qui contient (1) un polymère comportant un groupe acide sulfonique dans son unité répétitive, (2) un acide arylsulfonique, et (3) un sel de diazonium qui réagit avec la bilirubine pour produire de l'azobilirubine;
   (II) une couche absorbante capable d'absorber une partie de liquide en excès; et
   (III) un support qui transmet la lumière et qui est imperméable aux liquides.

2. Elément d'analyse selon la revendication 1, dans lequel ledit polymère contient les unités répétitives comportant un groupe acide sulfonique dans un rapport d'au moins 10 mol % sur la base de l'ensemble des unités répétitives constituant ledit polymère.

3. Elément d'analyse selon la revendication 1, dans lequel la teneur en ledit acide arylsulfonique est comprise dans la gamme de 7 à 70 fois la teneur en le sel de diazonium en rapport pondéral.

4. Elément d'analyse selon la revendication 1 ou 2, dans lequel ladite couche réactive poreuse contient de fines particules qui arrêtent la lumière.

5. Elément d'analyse selon la revendication 4, dans lequel lesdites fines particules qui arrêtent la lumière sont de fines particules de dioxyde de titane.

6. Elément d'analyse selon la revendication 1 ou 2, dans lequel ladite couche réactive poreuse comporte une matrice choisie dans le groupe formé par les étoffes tissées et les étoffes tricotées.

7. Elément d'analyse selon la revendication 1 ou 2, dans lequel ladite couche absorbante comprend un polymère hydrophile ayant un degré de gonflement dans l'eau à 30°C compris dans la gamme de 1,5 à 30 fois le volume d'origine.

8. Elément d'analyse selon la revendication 1 ou 2, dans lequel une couche destinée à empêcher la diffusion de la substance à analyser est disposée entre la couche réactive poreuse et la couche absorbante.

## Patentansprüche

1. Mehrschichtiges integrales analytisches Element zur quantitativen Bestimmung von Bilirubin in einer flüssigen Probe, **gekennzeichnet,** durch:
   (I) eine poröse Reagensschicht, die (1) ein Polymeres mit einer Sulfonsäuregruppe in seiner Repetiereinheit, (2) eine Arylsulfonsäure, und (3) ein Diazoniumsalz, das gegenüber Bilirubin und der Erzeugung von Azobilirubin aktiv ist, enthält;
   (II) eine absorbierende Schicht, die dazu imstande ist, einen überschüssigen Teil der Flüssigkeit zu absorbieren; und
   (III) einen flüssigkeitsundurchlässigen lichtdurchlässigen Träger.

2. Analytisches Element nach Anspruch 1, dadurch **gekennzeichnet,** daß das Polymere die Repetiereinheiten mit einer Sulfonsäuregruppe in einem Verhältnis von mindestens 10 mol%, bezogen auf die gesamten, das Polymere bildenden Repetiereinheiten, enthält.

3. Analytisches Element nach Anspruch 1, dadurch **gekennzeichnet,** daß der Gehalt der Arylsulfonsäure, bezogen auf das Gewicht, 7 bis 70mal so groß ist wie derjenige des Diazoniumsalzes.

4. Analytisches Element nach Anspruch 1 oder 2, dadurch **gekennzeichnet,** daß die poröse Reagensschicht lichtblockierende feine Teilchen enthält.

5. Analytisches Element nach Anspruch 4, dadurch **gekennzeichnet,** daß die lichtblockierenden feinen Teilchen feine Titandioxid-Teilchen sind.

6. Analytisches Element nach Anspruch 1 oder 2, dadurch **gekennzeichnet** daß die poröse Reagensschicht eine Matrix, ausgewählt aus der Gruppe, bestehend aus gewebtem Stoff und gewirktem Stoff, aufweist.

7. Analytisches Element nach Anspruch 1 oder 2, dadurch **gekennzeichnet,** daß die absorbierende Schicht ein hydrophiles Polymeres mit einem Quellungsgrad in Wasser bei 30°C, der 1,5 bis 30mal so groß ist wie das ursprüngliche Volumen, aufweist.

8. Analytisches Element nach Anspruch 1 oder 2, dadurch **gekennzeichnet,** daß eine Schicht zur Verhinderung der Diffusion des Analyten zwischen der porösen Reagensschicht und der absorbieren-

den Schicht vorgesehen ist.